# EUROPÄISCHE PATENTSCHRIFT

(11) **EP 1 165 579 B1**
(45) Veröffentlichungstag und Bekanntmachung des Hinweises auf die Patenterteilung: **22.03.2006**
(21) Anmeldenummer: 00909104.2
(22) Anmeldetag: 26.01.2000
(51) Int. Cl.: C07H 1/08, C12N 15/10, A61K 31/70

(54) **VERFAHREN ZUR HERSTELLUNG ENDOTOXINFREIER NUKLEINSÄUREN**
METHOD FOR PRODUCING ENDOTOXIN-FREE NUCLEIC ACIDS
TECHNIQUE PERMETTANT DE PRODUIRE DES ACIDES NUCLEIQUES DEPOURVUS D'ENDOTOXINES

(30) Priorität: 29.01.1999 DE 19903507
(43) Veröffentlichungstag der Anmeldung: 02.01.2002
(73) Patentinhaber: Xantos Biomedicine AG, 82152 Martinsried (DE)
(72) Erfinder: GRIMM, Stefan, 81241 München (DE); NEUDECKER, Frank, 80992 München (DE)
(74) Vertreter: Weiss, Wolfgang
(86) Internationale Anmeldenummer: PCT/EP2000/000564
(87) Internationale Veröffentlichungsnummer: WO 2000/044759

(56) Entgegenhaltungen:
- WO-A-89/07603
- WO-A-91/02740
- WO-A-93/08894
- WO-A-95/21177
- WO-A-95/21178
- WO-A-95/21179
- US-A- 4 833 239
- PRAZERES D M F ET AL: "Preparative purification of supercoiled plasmid DNA using anion-exchange chromatography" JOURNAL OF CHROMATOGRAPHY A,NL,ELSEVIER SCIENCE, Bd. 806, Nr. 1, 8. Mai 1998 (1998-05-08), Seiten 31-45, XP004121166 ISSN: 0021-9673
- LEVISON P R ET AL: "New approaches to the isolation of DNA by ion-exchange chromatography" JOURNAL OF CHROMATOGRAPHY A,NL,ELSEVIER SCIENCE, Bd. 827, Nr. 2, 11. Dezember 1998 (1998-12-11), Seiten 337-344, XP004153868 ISSN: 0021-9673
- LEVISON P R ET AL: "Recent developments of magnetic beads for use in nucleic acid purification" JOURNAL OF CHROMATOGRAPHY A,NL,ELSEVIER SCIENCE, Bd. 816, Nr. 1, 7. August 1998 (1998-08-07), Seiten 107-111, XP004145838 ISSN: 0021-9673
- PRAZERES D M F ET AL: "Large-scale production of pharmaceutical-grade plasmid DNA for gene therapy: problems and bottlenecks" TRENDS IN BIOTECHNOLOGY,NL,ELSEVIER, AMSTERDAM, Bd. 17, Nr. 4, April 1999 (1999-04), Seiten 169-174, XP004162836 ISSN: 0167-7799
- WANG: 'Purification of genomic DNA from human whole blood by isopropanol-fractionation with concentrated NaI and SDS' NUCLEIC ACIDS RESEARCH Bd. 22, Nr. 9, 1994, Seiten 1774 - 1775

## Beschreibung

Gegenstand der Erfindung ist ein Verfahren zur Isolierung und Reinigung von Nukleinsäuren und/oder Oligonukleotiden aus einer biologischen Probe, Verwendung der isolierten bzw. gereinigten Nukleinsäure bzw. Oligonukleotide zur Transfektion von Zellen sowie zur Herstellung eines Mittels zur Behandlung von genetisch bedingten Erkrankungen, eine für das Isolierungs- bzw. Reinigungsverfahren geeignete Zusammensetzung sowie die Verwendung von Kaliumacetat und ein Silicagel-ähnliches Trägermaterial zur Isolierung von endotoxinfreier oder an Endotoxin abgereicherter Nukleinsäuren und/oder Oligonukleotiden.

Die Qualität isolierter Nukleinsäuren gewinnt zunehmend an Bedeutung. Hochreine Nukleinsäure-Fraktionen, d.h. von denen möglichst sämtliche anderen Zellbestandteile, wie beispielsweise Endotoxine abgetrennt sind, spielen eine zentrale Rolle in der Gentherapie bzw. bei der Transfektion von Zellen eukaryontischen oder auch prokaryontischen Ursprungs. Demzufolge wurden in den vergangenen Jahren vermehrt Verfahren bzw. Maßnahmen publiziert, die die Isolierung von Nukleinsäuren aus biologischem Probenmaterial mit hohem Reinheitsgrad erlauben. Im wesentlichen kommen bei den bekannten Verfahren der Einsatz von Affinitäts- und/oder Anionenchromatographie-Materialien sowie nicht ionische Detergenzien oder auch verdünnte Lösungen höherer Alkohole zum Einsatz. Beispielsweise werden gemäß WO95/21177 die interessierenden Fraktionen einer Affinitätschromatographie oder einer Chromatographie an anorganischer Festphase, letzteres bevorzugt in Gegenwart eines nicht ionischen Detergenzes, zur Entfernung von Endotoxinen unterzogen und anschließend mittels Anionenaustauscherchromatographie weiter gereinigt. Ein solches zweistufiges Chromatographie-Verfahren ist jedoch zeit-und materialaufwendig und daher mehr von akademischem Wert. Nach einem anderen Verfahren (WO95/21178) ist ebenfalls zwingend eine aufwendige Anionenaustauscherchromatographie erforderlich, um Rückstände zuvor zugesetzter komplexer Salzlösung abzutrennen.

Darüber hinaus ist seit längerem bekannt, daß DNA-Plasmide aus komplexen biologischen Proben eukaryontischen oder prokaryontischen Ursprungs durch die Bindung an Silicagel in Gegenwart chaotroper Salze, wie beispielsweise Guanidiniumhydrochlorid isoliert werden können (M.A. Marko et al., Analyt. Biochem. 121, (1982) 382-287; EP 0 389 063). Diese Verfahren sind jedoch nicht für die Gewinnung endotoxinarmer bzw. endotoxinfreier Nukleinsäurefraktionen geeignet. So konnte beispielsweise gezeigt werden, daß die Maßnahmen gemäß Marko et al. (1982) zu einem Endotoxingehalt von mehr als 10.000 EU pro µg DNA führen. Eine solche endotoxinreiche DNA-Fraktion ist für die Transfektion von Zellen bei gentherapeutischen Anwendungen nicht geeignet.

Der Erfindung lag daher die Aufgabe zugrunde, ein Verfahren zur Herstellung von endotoxinfreien bzw. an Endotoxin abgereicherten Nukleinsäuren zur Verfügung zu stellen, wodurch die Nachteile bekannter Verfahren, wie insbesondere aufwendige Säulenmaterialien, vermieden werden.

Die Aufgabe wird gelöst durch ein Verfahren zur Isolierung und Reinigung von Nukleinsäuren und/oder Oligonukleotiden aus biologischen Proben, wobei die jeweilige biologische Probe aufgeschlossen wird, dabei nicht gelöste Zell-Bestandteile in einer wäßrigen Kaliumacetat-Lösung resuspendiert, gegebenenfalls vorhandene unlösliche Bestandteile, beispielsweise durch Zentrifugation, abtrennt, die flüssige Phase mit einer ein Detergenz enthaltenden Alkohol-Lösung vermischt und inkubiert werden. Die Lösung wird anschließend mit einem Silicagel-ähnlichem Trägermaterial in Kontakt gebracht, die wäßrige Phase wird möglichst quantitativ von dem die Nukleinsäuren bzw. Oligonukleotide bindendem Trägermaterial abgetrennt - beispielsweise durch Absaugen oder Zentrifugieren - und das Trägermaterial mit der DNA wird anschließend ausreichend gewaschen. Als Waschlösung kann eine Alkohol-Lösung oder - was sich als besonders vorteilhaft herausgestellt hat - Aceton verwendet werden. Abhängig von dem Volumen der Ausgangsprobe ist eine Inkubationszeit für den Kontakt mit dem Trägermaterial von 10 bis maximal 40 Minuten bei Raumtemperatur ausreichend; erfindungsgemäß reichen in der Regel ca. 20 Minuten aus.

Dem Fachmann sind Silicagel-ähnliche Trägermaterialien prinzipiell bekannt. Erfindungsgemäß hat sich insbesondere eine Suspension von Siliciumdioxid als geeignet erwiesen. Eine Siliciumoxid-Suspension, welche durch Zugabe von Säure (z. B. Salzsäure) zu einer wäßrigen Suspension von Siliciumdioxid hergestellt und anschließend autoklaviert wurde, ist für das erfindungsgemäße Verfahren besonders geeignet.

Die wäßrige Kaliumacetat-Lösung enthält Kaliumacetat bevorzugt in einem Konzentrationsbereich von ca. 1 bis 6 mol/l, wobei ein Bereich von 2 bis 4 mol/l und ein schwach saurer pH-Wert (ca. pH 4.5-6.8) erfindungsgemäß zu einer besonders hohen Qualität der Nukleinsäuren geführt hat.

Eine weitere vorteilhafte Ausführungsform des erfindungsgemäßen Verfahrens ist, wenn der Probe nach Zugabe der Kaliumacetat-Lösung zusätzlich ein oder mehrere RNA-verdauende Enzyme, wie beispielsweise RNAse A und/oder RNAse T1, zugesetzt werden. Es hat sich insbesondere bei größeren Präparationen als vorteilhaft erwiesen, wenn das bzw. die RNA-verdauende(n) Enzym(e) im gleichen Medium/Puffer zugegeben wird, in dem zuvor das Kaliumacetat-Salz zugesetzt wurde. Alternativ, und zwar gilt dies insbesondere bei kleineren Ansätzen, können die RNA-verdauenden Enzyme auch bereits während des Aufschließens der biologischen Probe, d.h. zusammen mit dem Lyse-Puffer (z. B. zusammen mit Puffer P1 in Beispiel 1.2), zugegeben werden. Werden mehrere RNA-verdauende Enzyme zugesetzt, können diese in beliebigen Verhältnissen oder auch zu gleichen Teilen vorhanden sein. Die Endkonzentration an RNA-verdauenden Enzymen in dieser Lösung beträgt in der Regel bis bzw. um ca. 150 µg/ml; aber auch höhere Enzymkonzentrationen haben das erfindungsgemäße Verfahren nicht nachteilig beeinflußt.

In der Regel ist erfindungsgemäß bereits eine Inkubation für den Enzymverdau von 5 bis 10 Minuten bei 4°C, gegebenenfalls zunächst bei Raumtemperatur, mit der Kaliumacetat-Lösung ausreichend; abhängig von der Menge des eingesetzten Probenmaterials kann jedoch auch entsprechend länger inkubiert werden.

Erfindungsgemäß geeignete, ein Detergenz enthaltende Alkohol-Lösungen sind insbesondere hochprozentige Lösungen höherer Alkohole, wie Isopropanol. Besonders vorteilhaft hat sich erfindungsgemäß erwiesen, wenn die Alkohol-Lösung nicht mit Wasser verdünnt ist, also nahezu zu 100% aus dem jeweiligen Alkohol besteht und zusätzlich ein oder mehrere ionische Detergenzien, und zwar in einer Konzentration von 0,5 bis 10% (w/v) enthält. Eine 100% Isopropanol-Lösung, welche ca. 1 bis 4% (w/v) SDS enthält, hat sich erfindungsgemäß als besonders geeignet erwiesen.

Der Aufschluß bzw. die Vorreinigung der biologischen Probe kann prinzipiell nach dem Fachmann bekannten Verfahren erfolgen. Alkalische Lysemaßnahmen sind erfindungsgemäß, insbesondere bei bakteriellen Wirtszellen, bevorzugt. Auf diese Weise können Proteinkomponenten und andere lösliche Bestandteile entfernt werden, bevor der Rückstand, der im wesentlichen Nukleinsäurekomponenten und andere nicht lösliche Zellbestandteile enthält, mit der Kaliumacetat- bzw. Alkohol/Detergenz-Lösung in Kontakt gebracht wird.

Mit dem erfindungsgemäßen Verfahren können Nukleinsäuren, wie beispielsweise Plasmid-DNA in hoher Qualität gewonnen werden, d.h. insbesondere mit einem Endotoxin-Gehalt von weniger als 100 EU/µg DNA, in der Regel von maximal 10 EU/µg DNA.

Insbesondere ist als überraschend anzusehen, daß die DNA nach alkalischer Lyse, ohne daß - wie im Stand der Technik beschrieben - die Zugabe chaotroper Substanzen erforderlich ist, mit hoher Effizienz an die Adsorptionsmatrix gebunden werden kann. Die Abwesenheit zugesetzter chaotroper Substanzen führt zu erheblichen Verbesserungen und Vereinfachungen bei der anschließenden Aufreinigungsprozedur der DNA bzw. der entsprechenden Transfektion von Zielzellen, und zwar sowohl bei Zellen eukaryontischen als auch prokaryontischen Ursprungs.

Darüber hinaus sind die nach dem erfindungsgemäßen Verfahren erhältlichen endotoxinfreien bzw. an Endotoxin abgereicherten Nukleinsäuren bzw. Oligonukleotide zur Herstellung von Mitteln zur Behandlung von genetisch bedingten Krankheiten geeignet

Ein weiterer Aspekt der Erfindung sind Mittel bzw. Zusammensetzungen zur Gewinnung von Plasmid-DNA aus entsprechenden Wirtszellen, bei denen es sich beispielsweise um Mikrotiterplatten oder Blöcke handelt, die gegebenenfalls Minisäulen zur Aufreinigung der Plasmid-DNA enthalten können.

Die erfindungsgemäßen Zusammensetzungen enthalten im wesentlichen eine wäßrige Kaliumacetat-Lösung sowie eine ein Detergenz enthaltende Alkohol-Lösung und ein Silicagelähnliches Trägermaterial. Darüber hinaus ist vorteilhaft, wenn eine für den Aufschluß einer biologischen Probe geeignete Lösung, insbesondere für die alkalische Lyse, vorhanden ist. Bevorzugte Ausführungsformen der Zusammensetzung sind, wenn die Kaliumacetat-Lösung das Salz in einem Konzentrationsbereich von ca. 1 bis 6 M, besonders bevorzugt von ca. 2 bis 4 M in einem schwach sauren Medium (pH ca. 4.5-6.8) aufweist, die Alkohol-Lösung Isopropanol mit ca. 0,5 bis 10% (w/v) eines ionischen Detergenzes, wie beispielsweise SDS enthält und/oder es sich bei dem Trägermaterial um eine wäßrige Suspension von Siliciumdioxid handelt.

### Abbildung 1

Endotoxin(Lipopolysaccharid, LPS)-Gehalt in verschiedenen DNA-Plasmid-Fraktionen nach Aceton-Waschung ((c), (d)) und SDS-Fällung ((b), (d)). Die Plasmid-DNA wurde durch Bindung an Siliciumoxid isoliert und anschließend mit Isopropanol ((a), (b)) oder Aceton ((c), (d)) gewaschen, mit oder ohne LPS-Fällung in Gegenwart von SDS (2,5% in Isopropanol). Der LPS-Gehalt wurde colorimetrisch bestimmt, nach den Angaben des Herstellers (Boehringer Ingelheim, Deutschland).
(a) Isopropanol/ohne SDS,
(b) Isopropanol/mit SDS,
(c) Aceton/ohne SDS,
(d) Aceton/mit SDS

Die folgenden Beispiele erläutern die Erfindung weiter:

### 1.1 Zellkultur und Transfektion

Babyhamster Nierenzellen (BHK) wurden in DMEM (Dulbecco Modified Eagle Medium) ergänzt mit 5% fötalem Kälberserum (Sigma, Deisenhofen, Deutschland) in einer befeuchteten 5% CO₂-Atmosphäre kultiviert. Für Transfektionen wurden die Zellen in 24-Loch-Platten gegeben und mit 2 µg Plasmid-DNA nach der Calciumphosphat-Copräzipitationsmethode wie von Roussel et al. (Mol. Cell. Biol. 4 (1984), 1999-2009) beschrieben transfiziert. Hierfür wurden 25 µl DNA Lösung mit 25 µl 2 x HBS: 274 mM NaCl, 10 mM KCI, 40 mM HEPES, 1.4 mM Na₂PO₄, pH 6,9 bei 4°C in einer 96-Loch-Platte mit einem 12-Kanal-Pipettierautomaten (Eppendorf, Hamburg, Deutschland) vermischt. Nach Zugabe von 20 µl einer 0,25 M CaCl₂-Lösung (4°C) und Mischen wurden 38 µl nach Inkubation für 25 min bei Raumtemperatur auf die Zellen gegeben.

Entsprechende Aliquots wurden in Löchern von 96-Loch-Blöcken (Qiagen, Hilden, Deutschland) in 900 µl TB-Medium inokuliert und für ca. 30 Stunden unter Schütteln bei 300 Upm kultiviert (37°C). Nach Identifizierung eines positiven Pools wurde die DNA zur Bestätigung des Ergebnisses erneut transfiziert. Die verbleibende DNA wurde zur Transformation von Bakterien für eine Plasmidisolierung im großen Maßstab verwendet.

### 1.2 Plasmidisolierung mit Säulen

96-Loch-Blöcke (Qiagen, Hilden, Deutschland) mit Bakterien wurden für 5 min bei 3000 g (Sigma Zentrifugen, Osterode am Harz, Deutschland) zentrifugiert. Der Überstand wurde dekantiert und die Blöcke wurden für 2 bis 3 min umgekehrt auf saugfähiges Papiertuch gebracht. Dann wurden 170 µl Puffer P1 (50 mM Tris-HCl/10 mM EDTA pH 8,0, 4°C) zugegen und die Bakterienpellets wurden durch vollständige Vortexbehandlung für 10 bis 20 min resuspendiert. Nach Zugabe von 170 µl Puffer P2 (200 mM NaOH, 1% SDS) wurde der Block mit Folie abgedichtet, umgedreht und für 5 min bei Raumtemperatur inkubiert. Die Lyse wurde durch Zugabe von 170 µl von 4°C kaltem Puffer P3 (3 M Kaliumacetat pH 5,5, 4°C) beendet. Dann wurden 10 µl RnaseA-Lösung (1,7 mg/ml) zugegeben, für 5 min bei Raumtemperatur und dann bei -20°C inkubiert und erneut für 10 min bei 6000 Upm zentrifugiert. Der Überstand wurde in neue Blöcke dekantiert und 100 µl Puffer P4 (2,5% (w/v) SDS in Isopropanol) wurden zugegeben. Der Block wurde einer Vortexbehandlung für 5 min unterzogen und zuerst für 15 min bei 4°C und dann für 15 min bei 20°C inkubiert. Die Blöcke wurden für 10 min bei 6000 Upm zentrifugiert und der Überstand wurde in eine Anordnung von 96 Säulen (Qiagen) in entsprechend zugeschnittene 96-Loch-Platten hergestellt worden war. Diese Platten wurden in Vakuumkammern (Qiagen) gestellt. Dann wurden 150 µl Siliciumoxid-Suspension zugegeben und für 20 min bei Raumtemperatur inkubiert (die Siliciumoxid-Suspension wurde hergestellt durch Zugabe von 150 µl HCl (37%) zu 250 ml einer Suspension von 50 mg/ml SiO₂ (Sigma) und anschließendes Autoklavieren).

Nach Anlegen von Vakuum wurden die Säulen zweimal mit 600 µl Aceton (-20°C) gewaschen. Die 96-Loch-Säulenplatte wurde auf eine 96-Loch-Mikrotiterplatte gegeben und für 4 min bei 6000 Upm zentrifugiert. Die Säulenplatte wurde zuerst für 5 min bei 37°C und dann für 5 min in einer Vakuumkammer getrocknet. Dann wurde sie auf eine weitere Mikrotiterplatte gestellt. 70 µl bidestilliertes H₂O (60°C) wurden zugegeben und dann erfolgte eine Zentrifugation für 3 min bei 6000 Upm. Die Mikrotiterplatte wurde bei -20°C aufbewahrt.

### 1.3 Plasmidisolierung ohne Säulen

Das Verfahren erfolgte bis zur Zugabe des Puffers P4 wie unter Punkt 1.2 beschrieben. Dann wurde der Überstand nach Zentrifugation für 10 min bei 6000 Upm in 96-Loch-POM-Mikrotiterblöcke (POM=Polyoxymethlen) gegeben, 150 µl Siliciumoxid-Suspension wurden zugegeben und für 20 min bei Raumtemperatur inkubiert. Die Platten wurden für 5 min bei 6000 Upm zentrifugiert. Der Überstand wurde sorgfältig dekantiert und 400 µl Aceton (-20°C) wurden zugegeben. Die Platten wurden erneut einer Vortexbehandlung (30 sec) unterzogen und für 3 min bei 6000 Upm zentrifugiert. Dieser Acetonwaschvorgang wurde einmal wiederholt. Die Platten wurden zuerst bei Raumtemperatur für 5 min und dann für 5 min in einer Vakuumkammer getrocknet. Die Pellets wurden in 75 µl Wasser (60°C) resuspendiert und bei 6000 Upm und 4°C 10 min zentrifugiert. Der Überstand wurde in einer 96-Loch-Mikrotiterplate bei -20°C aufbewahrt.

### 2. Ergebnisse

Aus den Bakterienkulturen wurde Plasmid-DNA unter Verwendung von Minisäulen (s. Punkt 1.2) isoliert. Ein entsprechendes Protokoll ohne Säulen ist unter Punkt 1.3 beschrieben.

Für den Transfektionsschritt ist es wichtig, Plasmid-DNA sehr hoher Reinheit zu erhalten. Hierzu wurde Siliciumdioxid als Bindematrix für Plasmid-DNA verwendet. Die Bindung von DNA an Siliciumdioxid in Anwesenheit chaotroper Substanzen ist bekannt (Vogelstein und Gillespie, Proc. Natl. Acad. Sci. USA 76 (1979), 615-619). Überraschenderweise wurde jedoch festgestellt, daß selbst in Abwesenheit einer zugefügten chaotropen Substanz wie etwa Guanidinhydrochlorid die Plasmid-DNA mit ausreichender Kapazität an Siliciumdioxid bindet. Nach anschließendem Waschen in Aceton, gegebenenfalls unter Zusatz von SDS, konnte Plasmid-DNA in hervorragender Qualität - entsprechend einer Reinigung über einen Cäsiumchloridgradienten - erhalten werden. Üblicherweise wurden etwa 10 µg Plasmid-DNA aus 900 µl LB-Medium mit einer OD260/280 von mehr als 1,8 erhalten, wovon 90% in der supercoiled Form vorlagen.

### 3. Vergleich mit demStand der Technik

Versuch A: Bakterienkultur: *E.coli* HB101 pCMVbetaSportGAL, OD₆₈₀/ml ca. 3.3

Es wurden in einem Doppelansatz je 1,8 ml Bakterienkultur mit dem High Pure Plasmid Isolation Kit (Boehringer Mannheim, Cat. No. 1 754 777), welcher ein glasartiges Trägermaterial und ein stark chaotropes Salz enthält, aufgearbeitet und je 1,8 ml Bakterienkultur gemäß dem erfindungsgemäßen Verfahren prozessiert.

Das Ergebnis stellt sich wie folgt dar:

| Ausbeute OD₂₆₀nm: | Endotoxin-Gehalt (LAL Test) |
|---|---|
| High Pure 1 : 9,0 µg/100 µl endotoxinfreies Wasser | 214 EU/µg Plasmid |
| High Pure 2 : 8,6 µg/100 µl endotoxinfreies Wasser | 240 EU/µg Plasmid |
| Erfindung 1:11,00 µg/100 µl endotoxinfreies Wasser | 1,41 EU/µg Plasmid |
| Erfindung 2: 10,35 µg/100 µl endotoxinfreies Wasser | 4,65 EU/µg Plasmid |

### Vorgehen gemäß dem erfindungsgemäßen Verfahren mit High Pure Filter Tube:

Die Bakterienkultur wurde für 30 sec. bei 13000 upm zentrifugiert und Überstand abgenommen.

Das Zellsediment von 1.8 ml Bakterienkultur wurde wie folgt weiterbehandelt:
1. Resuspendieren in 250 µl 50 mM Tris-HCl/10 mM EDTA, 100 µg RNase (DNase frei), pH 8.0, 4°C.
2. 250 µl 0.2 M NaOH, 1% SDS zugeben und das Gefäß 5-10 x invertieren, 5 min. bei RT.
3. 250 µl 3 M K-Acetat, pH 5.5 zugeben (4°C) und das Gefäß 5-10 x invertieren, 5 min. auf Eis inkubieren.
4. 10 min. bei maximaler Geschwindigkeit in einer Tischzentrifuge zentrifugieren (14000 rpm), Überstand abnehmen und 0,2 vol. (ca. 150 µl) 2.5% SDS in Isopropanol (z. B. 7 ml Isopropanol und 1 ml 20% SDS) zugeben und kurz vortexen, 15 min. bei 4°C inkubieren und anschließend 15 min. bei -20°C inkubieren.
5. 10 min. bei maximaler Geschwindigkeit in einer Tischzentrifuge zentrifugieren (14000 upm), Überstand abnehmen.
6. Überstand in High Pure Filter Tube pipettieren und 20 min. bei RT inkubieren.
7. 30 sec. bei maximaler Geschwindigkeit in einer Tischzentrifuge zentrifugieren (14000 upm), Durchlauf verwerfen und Filter Tube 2 x mit 700 µl eiskaltem Aceton waschen (Zwischen den Waschschritten 30 sec. bei 14000 upm zentrifugieren).
8. Nach dem letzten Waschschritt nochmals 30 sec. bei 14000 upm zentrifugieren, um das Vlies zu trocknen.
9. DNA durch Zugabe von 100 µl endotoxinfreiem Wasser und Inkubation für 10 min. bei RT eluieren. Um die DNA zu gewinnen wird 30-60 sec. bei voller Zentrifugationsgeschwindigkeit zentrifugiert.

Versuch B: Bakterienkultur: *E.coli* JM109pCMVbetaSportGal OD₅₈₀/ml 2.37

| Probe | Methode | Modifikation | Ausbeute [µg/100µg] | Endotoxin [EU/µg] |
|---|---|---|---|---|
| 1 und 2 | High Pure | | 9,3 / 9,3 | 371,7 |
| 3 und 4 | High Pure | 20 min. auf Vlies inkubiert 10 min. vor Elution inkubiert | 12,8 / 12, 2 | 2,18 |
| 5 und 6 | Erfindung | Ohne Inkubationen | 12,2 / 12,6 | 0,63 |

### Ergebnis:

- Das erfindungsgemäße Verfahren zeigt eine ca. 100fache Reduktion des Endotoxinwertes.
- Ferner führt das erfindungsgemäße Verfahren mit schneller Durchzentrifugation zur gleichen Ausbeute wie mit Inkubation auf Vlies, damit kann die Zeit der Reinigung nun mit ca. 70 min. angegeben werden. Das erfindungsgemäße Verfahren mit schneller Durchzentrifugation zeigt darüber hinaus einen niedrigeren Endotoxinwert als nach der Inkubation auf Vlies.

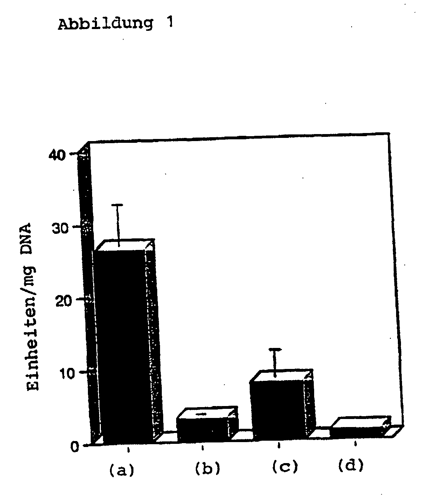

## Patentansprüche

1. Verfahren zur Isolierung und Reinigung von Nukleinsäuren und /oder Oligonukleotiden aus einer biologischen Probe,
**dadurch gekennzeichnet,**
**dass**
- die biologische Probe aufgeschlossen, Proteinkomponenten und andere unlösliche Bestandteile abgetrennt werden,
- zum Rückstand eine wässrige Lösung an Kaliumacetat gegeben wird und nicht lösliche Bestandteile abgetrennt werden,
- die mit Kaliumacetat versetzte Lösung mit einer ein Detergenz enthaltenden Alkohol-Lösung vermischt und inkubiert wird,
- der erhaltene Überstand mit einem Silicagel-ähnlichen Trägermaterial in Kontakt gebracht und inkubiert wird, und
- aus der löslichen Fraktion die gereinigten Nukleinsäuren und/oder Oligonukleotide isoliert werden.

2. Verfahren nach Anspruch 1,
**dadurch gekennzeichnet,**
**dass** es sich bei der Alkohol-Lösung um eine Mischung von Isopropanol mit einem ionischen Detergenz handelt.

3. Verfahren nach Anspruch 1 oder 2,
**dadurch gekennzeichnet,**
**dass** die Alkohol-Lösung ein oder mehrere ionische Detergenzien in einer Konzentration von 0,5 bis 10 % (w/v) in 100 %igem Alkohol enthält.

4. Verfahren nach Anspruch 1 bis 3,
**dadurch gekennzeichnet,**
**dass** eine wässrige Lösung, die 1 bis 6 M Kaliumacetat enthält, verwendet wird.

5. Verfahren nach Anspruch 4,
**dadurch gekennzeichnet,**
**dass** die Lösung 2 bis 4 M Kaliumacetat enthält.

6. Verfahren nach einem der Ansprüche 1 bis 5,
**dadurch gekennzeichnet,**
**dass** als Silicagel-ähnliches Trägermaterial eine Suspension von Siliciumoxid eingesetzt wird.

7. Verfahren nach einem der Ansprüche 1 bis 6,
**dadurch gekennzeichnet,**
**dass** das Silicagel-ähnliche Trägermaterial mit Aceton nachgewaschen wird.

8. Verfahren nach einem der Ansprüche 1 bis 7,
**dadurch gekennzeichnet,**
**dass** Plasmid-DNA mit einem Endotoxin-Gehalt von weniger als 100 EU/µg erhalten wird.

9. Verfahren nach Anspruch 8,
**dadurch gekennzeichnet,**
**dass** der Endotoxin-Gehalt maximal 10 EU/µg Plasmid-DNA beträgt.

10. Verfahren nach einem der Ansprüche 1 bis 9,
zusätzlich umfassend den Schritt der Transfektion von eukaryontischen oder prokaryontischen Zellen mit den gewonnenen Nukleinsäuren und/oder Oligonukleotiden.

11. Verfahren nach einem der Ansprüche 1 bis 9,
zusätzlich umfassend den Schritt der Herstellung eines Mittels zur Behandlung von genetisch bedingten Erkrankungen unter Verwendung der gewonnenen Nukleinsäuren und/oder Oligonukleotiden.

12. Zusammensetzung, enthaltend folgende Komponenten:
- mindestens eine für den Aufschluss einer biologischen Probe geeignete Lösung,
- eine wässrige Kaliumacetat-Lösung,
- eine Detergenz/Alkohol-Mischung, und
- ein Silicagel-ähnliches Trägermaterial.

13. Zusammensetzung nach Anspruch 12,
**dadurch gekennzeichnet,**
**dass** folgende Komponenten enthalten sind:
- eine für die alkalische Lyse von biologischem Probenmaterial geeignete Lösung,
- eine Salz-Lösung, die 1 bis 6 M Kaliumacetat enthält,
- eine Alkohol-Lösung enthaltend 0,5 bis 10 % (w/v) SDS in 100 %igem Isopropanol und
- ein Silicagel-ähnliches Trägermaterial.

14. Zusammensetzung nach Anspruch 12 oder 13,
**dadurch gekennzeichnet,**
**dass** eine Suspension von Siliciumoxid als Trägermaterial enthalten ist.

15. Verwendung von Kaliumacetat als Fällungsreagenz zur Abtrennung von nicht löslichen Bestandteilen aus einer DNA enthaltenden Lösung in einerm Verfahren nach einem der Ansprüche 1 bis 11 bzw. aus einer vorgereinigten biologischen Probe in einem Verfahren nach einem der Ansprüche 1 bis 11.

16. Reagenzienkit umfassend eine Zusammensetzung nach einem der Ansprüche 12, 13 oder 14.

## Claims

1. Method for isolating and purifying nucleic acids and/or oligonucleotides from a biological sample,
**characterized in that**
- the biological sample is disrupted, protein components and other insoluble components are removed,
- an aqueous solution of potassium acetate is added to the residue and non-soluble components are removed,
- the potassium acetate-containing solution is mixed and incubated with an alcoholic solution containing a detergent,
- the supernatant obtained is contacted and incubated with a silica gel-like support material, and
- the purified nucleic acids and/or oligonucleotides are isolated from the soluble fraction.

2. Method according to Claim 1, **characterized in that** the alcoholic solution is a mixture of isopropanol with an ionic detergent.

3. Method according to Claim 1 or 2, **characterized in that** the alcoholic solution contains one or more ionic detergents at a concentration of 0.5 to 10% (w/v) in 100% strength alcohol.

4. Method according to Claims 1 to 3, **characterized in that** an aqueous solution containing 1 to 6 M potassium acetate is used.

5. Method according to Claim 4, **characterized in that** the solution contains 2 to 4 M potassium acetate.

6. Method according to any of Claims 1 to 5, **characterized in that** the silica gel-like support material used is a suspension of silicon oxide.

7. Method according to any of Claims 1 to 6, **characterized in that** the silica gel-like support material is washed with acetone.

8. Method according to any of Claims 1 to 7, **characterized in that** plasmid DNA with an endotoxin content of less than 100 EU/µg is obtained.

9. Method according to Claim 8, **characterized in that** the endotoxin content is not more than 10 EU/µg of plasmid DNA.

10. Method according to any of Claims 1 to 9 additionally comprising the step of transfecting eukaryotic or prokaryotic cells using the nucleic acids and/or oligonucleotides obtained.

11. Method according to any of Claims 1 to 9 additionally comprising the step of producing an agent for the treatment of genetic disorders using the nucleic acids and/or oligonucleotides obtained.

12. Composition comprising the following components:
- at least one solution suitable for the disruption of a biological sample,
- an aqueous potassium acetate solution,
- a solution of detergent/alcohol, and
- a silica gel-like support material.

13. Composition according to Claim 12, **characterized in that** the following components are included:
- a solution suitable for alkaline lysis of biological sample material,
- a salt solution containing 1 to 6 M potassium acetate,
- an alcoholic solution containing 0.5 to 10% (w/v) SDS in 100% strength isopropanol and
- a silica gel-like support material.

14. Composition according to Claim 12 or 13, **characterized in that** the support material included is a suspension of silicon dioxide.

15. Use of potassium acetate as a precipitant for removing non-soluble components from a DNA-containing solution in a method according to any of Claims 1 to 11 or from a pre-purified biological sample in a method according to any of Claims 1 to 11.

16. Reagent kit comprising a composition according to any of Claims 12, 13 and 14.

## Revendications

1. Procédé pour isoler et purifier des acides nucléiques et/ou des oligonucléotides à partir d'un échantillon biologique,
**caractérisé en ce que**
- l'échantillon biologique est désagrégé, les composants protéiques et les autres constituants insolubles sont séparés,
- une solution aqueuse d'acétate de potassium est ajoutée au résidu et les constituants non solubles sont séparés,
- la solution additionnée d'acétate de potassium est mélangée avec une solution alcoolique contenant un détergent et incubée,
- le surnageant obtenu est mis en contact avec un support analogue à du gel de silice et incubé, et
- les acides nucléiques et/ou oligonucléotides purifiés sont isolés à partir de la fraction soluble.

2. Procédé selon la revendication 1
**caractérisé**
**en ce que** la solution alcoolique est un mélange d'isopropanol et d'un détergent ionique.

3. Procédé selon la revendication 1 ou 2
**caractérisé**
**en ce que** la solution alcoolique contient un ou plusieurs détergents ioniques en une concentration de 0,5 à 10 % (m/v) dans de l'alcool à 100 %.

4. Procédé selon les revendications 1 à 3
**caractérisé**
**en ce qu'**une solution aqueuse qui contient de l'acétate de potassium 1 à 6 M est utilisée.

5. Procédé selon la revendication 4
**caractérisé**
**en ce que** la solution contient de l'acétate de potassium 2 à 4 M.

6. Procédé selon l'une des revendications 1 à 5
**caractérisé**
**en ce qu'**une suspension d'oxyde de silicium est utilisée comme support analogue à du gel de silice.

7. Procédé selon l'une des revendications 1 à 6
**caractérisé**
**en ce que** le support analogue à du gel de silice est lavé avec de l'acétone.

8. Procédé selon l'une des revendications 1 à 7
**caractérisé**
**en ce que** de l'ADN plasmidique ayant une teneur en endotoxines inférieure à 100 UE/µg est obtenu.

9. Procédé selon la revendication 8
**caractérisé**
**en ce que** la teneur en endotoxines est au maximum de 10 UE/µg d'ADN plasmidique.

10. Procédé selon l'une des revendications 1 à 9
comprenant en outre l'étape de transfection de cellules eucaryotes ou procaryotes avec les acides nucléiques et/ou oligonucléotides obtenus.

11. Procédé selon l'une des revendications 1 à 9
comprenant en outre l'étape de production d'un agent pour le traitement de maladies génétiques au moyen des acides nucléiques et/ou oligonucléotides obtenus.

12. Composition contenant les composants suivants :
- au moins une solution appropriée pour la désagrégation d'un échantillon biologique,
- une solution aqueuse d'acétate de potassium,
- un mélange détergent/alcool et
- un support analogue à du gel de silice.

13. Composition selon la revendication 12
**caractérisée**
**en ce que** les composants suivants sont contenus :
- une solution appropriée pour la lyse alcaline d'un échantillon biologique,
- une solution de sel qui contient de l'acétate de potassium 1 à 6 M,
- une solution alcoolique contenant 0,5 à 10 % (m/v) de SDS dans de l'isopropanol à 100 % et
- un support analogue à du gel de silice.

14. Composition selon la revendication 12 ou 13
**caractérisée**
**en ce qu'**une suspension d'oxyde de silicium est contenue comme support.

15. Utilisation d'acétate de potassium comme réactif de précipitation pour la séparation des constituants non solubles d'une solution contenant de l'ADN dans un procédé selon l'une des revendications 1 à 11 ou d'un échantillon biologique prépurifié dans un procédé selon l'une des revendications 1 à 11.

16. Kit de réactifs comprenant une composition selon l'une des revendications 12, 13 ou 14.
